# EUROPEAN PATENT SPECIFICATION

(11) **EP 2 583 973 B1**
(45) Date of publication and mention of the grant of the patent: **21.03.2018**
(21) Application number: 11798094.6
(22) Date of filing: 20.06.2011
(51) Int. Cl.: C07K 1/16, C07K 1/18, C07K 1/20, C07K 1/22, C07K 16/00

(54) **METHOD FOR PURIFYING PROTEIN USING AMINO ACID**
VERFAHREN ZUR REINIGUNG VON PROTEINEN MIT AMINOSÄUREN
PROCÉDÉ DE PURIFICATION D'UNE PROTÉINE UTILISANT UN ACIDE AMINÉ

(30) Priority: 09.02.2011 US 201161441051 P; 21.06.2010 US 356899 P
(43) Date of publication of application: 24.04.2013
(60) Divisional application: 17186833.4
(73) Proprietor: Kyowa Hakko Kirin Co., Ltd., Tokyo 100-8185 (JP)
(72) Inventor: ISHIHARA, Takashi, 100-1, Hagiwara-machi Takasaki-shi (JP)
(74) Representative: Hoffmann Eitle
(86) International application number: PCT/JP2011/064074
(87) International publication number: WO 2011/162210

(56) References cited:
- WO-A1-2010/019493
- WO-A1-2010/056550
- JP-A- 2009 522 580
- US-A- 5 429 746
- US-A1- 2005 176 109
- US-A1- 2006 030 696
- US-A1- 2006 257 972
- US-A1- 2007 054 390
- US-A1- 2008 064 861
- US-A1- 2008 312 425
- US-A1- 2009 162 372
- US-A1- 2009 286 283
- US-A1- 2009 318 674
- JUNGBAUER A ET AL: "Pilot scale production of a human monoclonal antibody against human immunodeficiency virus", JOURNAL OF BIOCHEMICAL AND BIOPHYSICAL METHODS, AMSTERDAM, NL, vol. 19, no. 2/03, 1 January 1989 (1989-01-01), pages 223-240, XP002114282, ISSN: 0165-022X, DOI: 10.1016/0165-022X(89)90029-8
- ARAKAWA,T. ET AL.: 'The effects of arginine on protein binding and elution in hydrophobic interaction and ion-exchange chromatography' PROTEIN EXPR.PURIF. vol. 54, no. 1, 2007, pages 110 - 116, XP022056642
- JUNGBAUER A ET AL: "Pilot scale production of a human monoclonal antibody against human immunodeficiency virus", JOURNAL OF BIOCHEMICAL AND BIOPHYSICAL METHODS, AMSTERDAM, NL, vol. 19, no. 2/03, 1 January 1989 (1989-01-01), pages 223-240, XP002114282, ISSN: 0165-022X, DOI: 10.1016/0165-022X(89)90029-8

## Description

### TECHNICAL FIELD

The present invention relates to a general protein purification method. In particular, the present invention relates to a purification method of antibodies by chromatography.

### BACKGROUND ART

Development of genetic recombination technologies has provided a variety of protein pharmaceuticals. In particular, numerous antibody pharmaceuticals have been recently developed and commercialized. In addition, the large-scale, economic purification of these protein pharmaceuticals has become a more important issue in biopharmaceutical industry.

Generally, these protein pharmaceuticals are produced by culturing recombinant cells into which a vector including an objective protein gene is inserted. The culture fluid includes impurities such as various medium-derived ingredients, cell by-products from cells or the like, in addition to the objective protein. Thus, it is very difficult and challenging to isolate and purify the protein to meet purity requirements for protein pharmaceuticals, and further combine the above described large-scale production and economic efficiency.

In general, the purification method of the objective protein is carried out by a combination of different modes of chromatography. This method is, for example, a separation method of separating a protein mixture, based on charge, hydrophilicity, or size. Several different chromatographic resins can be used in each of these methods, and it is also possible to accurately adjust them to a purification plan for a specific protein.

The essence of each of the separation methods is that proteins are allowed to pass a long column at different speeds by increasing a physical separation to pass the column faster, or that selective adhesion to a separation medium is effected by carrying out different elutions with different solvents. In some cases, the objective protein is separated from impurities when the impurities adhere to the column resin quite well and the objective protein does not adhere thereto, i.e., the objective protein is in the "flow-through".

In particular, when the objective protein is an antibody obtained from animal cells as a host, Protein A or Protein G affinity chromatography is carried out by utilizing a binding specificity of Protein A or Protein G to the Fc chain of IgG or the like, and then a combination of cation exchange chromatography, anion exchange chromatography, hydroxyapatite chromatography, hydrophobic chromatography, gel filtration chromatography, mixed mode chromatography or the like is carried out to achieve purification.

For example, an antibody-containing aqueous culture medium obtained from a mammalian cell culture is applied to the Protein A or Protein G column chromatography to adsorb the antibodies to the column resin. Subsequently, the antibodies are eluted using an acidic solution, and the obtained eluate is sequentially applied to an ion exchange chromatography, and a size exclusion column chromatography to acheive purification (Patent Document1).

Further, there is a report that polymerization is inhibited by using an amino acid, arginine, as a buffer additive for Protein A affinity chromatography, ion exchange chromatography, hydrophobic chromatography (Non-Patent Documents 1 and 2).

### CITATION LIST

### PATENT DOCUMENT

[Patent Document 1] A published Japanese translation of a PCT application-5-504579

### NON-PATENT DOCUMENTS

[Non-Patent Document 1] Protein Expression and Purification 36(2004)244-248
[Non-Patent Document 2] Protein Expression and Purification 54(2007)110-116

### SUMMARY OF THE INVENTION

### PROBLEMS TO BE SOLVED BY THE INVENTION

However, determination of the chromatographic conditions or combination of different chromatographic methods takes much time, labor and cost, and is complicated. Additionally, it is not assured that the purification can be carried out to meet the required purity for protein pharmaceuticals.

Additionally, the way and purpose of use of technologies described in Non-Patent Documents 1 and 2 are limited, i.e., only arginine is used as the amino acid to be added and the effect is only the inhibition of polymerization, and thus it is hard to say that the technologies are methods for purifying proteins that can be achieve high purity.

Therefore, an object of the present invention is to provide a method for purifying a protein capable of providing high purity by more certainly removing impurities than the conventional purification methods. Further, another object of the present invention is to provide a method for purifying a protein capable of reducing cost or labor, because cost or labor can be a great problem in industrial-scale purification.

### MEANS FOR SOLVING THE PROBLEMS

The present inventors have made extensive studies to solve the problems and surprisingly, found that an objective protein can be purified with higher purity than the conventional methods by including an amino acid as an ingredient of a buffer solution or a part thereof used in at least one chromatographic process in a method for purifying a protein including one or more chromatographic processes, thereby completing the present invention.

The present invention provides:
[1]. A method for purifying a protein to reduce the amount of polymers and host cell proteins, comprising one or more chromatographic processes, wherein the one or more chromatographic processes comprises a Protein A affinity chromatographic process, and wherein glycine is included as the ingredient itself of the elution buffer used in the Protein A affinity chromatographic process, the content of the glycine in the elution buffer being 100 mM, and the pH of the elution buffer being from 3.2-3.4.
[2]. The purification method according to [1], wherein the one or more chromatographic processes further comprises a cation exchange chromatographic process.
[3]. The purification method according to [2], wherein an amino acid is included as an ingredient of a buffer solution or a part thereof used in the cation exchange chromatographic process.
[4]. The purification method according to any one of [1] to [3], which further comprises, as the chromatographic process, one or more chromatographic processes selected from an anion exchange chromatographic process, a gel filtration chromatographic process, a hydrophobic chromatographic process, a hydroxyapatite chromatographic process, and a mixed mode chromatographic process.
[5]. The purification method according to any one of [2] to [4], wherein the cation exchange chromatographic process is carried out subsequently to the Protein A affinity chromatographic process.
[6]. The purification method according to [5], wherein an anion exchange chromatographic process is further subsequently carried out.
[7]. The purification method according to [4], wherein the cation exchange chromatographic process is carried out subsequently to the Protein A affinity chromatographic process and an anion exchange chromatographic process.
[8]. The purification method according to any one of [2] to [7], wherein an amino acid included as an ingredient of a buffer solution or a part thereof used in the cation exchange chromatographic process is an amino acid selected from glutamic acid, histidine, and glutamine.
[9]. The purification method according to any one of [1] to [8], wherein a salt is added to a protein-containing sample that is loaded onto the Protein A affinity chromatography in the Protein A affinity chromatographic process.
[10]. The purification method according to any one of [2] to [9], wherein a ratio of host cell proteins in the purification product after completion of the cation exchange chromatographic process is less than 100 ng/mg.
[11]. The purification method according to any one of [2] to [10], wherein a ratio of polymers in the purification product after completion of the cation exchange chromatographic process is less than 1.5%.
[12]. A method for purifying a protein to reduce the amount of polymers and host cell proteins according to any one of [2] to [11], wherein an amino acid included in a pharmaceutical formulation of a medicinal product comprising the protein purified by the purification method according to any one of [2] to [11] as an active ingredient is identical to an amino acid included as an ingredient of the buffer solution or a part thereof used in the cation exchange chromatographic process according to any one of [2] to [11].

That is, the present disclosure provides (1) to (16) below.
(1). A method for purifying a protein, comprising one or more chromatographic processes, wherein an amino acid is included as an ingredient of a buffer solution or a part thereof used in at least one chromatographic process.
(2). The purification method described in (1), wherein an amino acid is included as an ingredient of a buffer solution or a part thereof used in a cation exchange chromatographic process.
(3). The purification method described in (2), which further comprises, as the chromatographic process, a Protein A affinity chromatographic process and one or more chromatographic processes selected from an anion exchange chromatographic process, a gel filtration chromatographic process, a hydrophobic chromatographic process, a hydroxyapatite chromatographic process, and a mixed mode chromatographic process.
(4). The purification method described in (2), wherein the cation exchange chromatographic process is carried out subsequent to the Protein A affinity chromatographic process.
(5). The purification method described in (4), wherein the anion exchange chromatographic process is further subsequently carried out.
(6). The purification method described in (3), wherein the cation exchange chromatographic process carried out subsequent to the Protein A affinity chromatographic process and the anion exchange chromatographic process.
(7). The purification method described in any one of (4) to (6), wherein an amino acid is included as an ingredient of a buffer solution or a part thereof used in the Protein A affinity chromatographic process.
(8). The purification method described in any one of (4) to (7), wherein the buffer solution used in the Protein A affinity chromatographic process is a buffer solution which comprises glycine as an ingredient of the buffer solution or a part thereof.
(9). The purification method described in any one of (3) to (8), wherein an amino acid included as an ingredient of a buffer solution or a part thereof used in the cation exchange chromatographic process is an amino acid selected from glutamic acid, histidine, and glutamine.
(10). The purification method described in any one of (4) to (9), wherein an amino acid included as an ingredient of a buffer solution or a part thereof used in the Protein A affinity chromatographic process is an amino acid selected from glycine and threonine.
(11). The purification method described in any one of (4) to (10), wherein a salt is added to a protein-containing sample that is loaded onto the Protein A affinity chromatography in the Protein A affinity chromatographic process.
(12). The purification method described in any one of (2) to (11), wherein a ratio of host cell proteins in the purification product after completion of the cation exchange chromatographic process is less than 100 ng/mg.
(13). The purification method described in any one of (2) to (12), wherein a ratio of polymers in the purification product after completion of the cation exchange chromatographic process is less than 1.5%.
(14). A method for purifying a protein, wherein an amino acid included in a pharmaceutical formulation of a medicinal product comprising the protein purified by the purification method described in any one of (3) to (13) as an active ingredient is identical to an amino acid included as an ingredient of the buffer solution or a part thereof used in the cation exchange chromatographic process described in any one of (3) to (13).
(15). The purification method described in (2), which further comprises, as the chromatographic process, at least one chromatographic processes selected from an anion exchange chromatographic process, a gel filtration chromatographic process, a hydrophobic chromatographic process, a hydroxyapatite chromatographic process, and a mixed mode chromatographic process.
(16). The purification method described in (1), which further comprises, as the chromatographic process, a chromatographic process selected from an anion exchange chromatographic process, a gel filtration chromatographic process, a hydrophobic chromatographic process, a hydroxyapatite chromatographic process, and a mixed mode chromatographic process.

### EFFECT OF THE INVENTION

An objective protein such as an antibody can be purified by the method for purifying a protein according to the present invention to greatly reduce the amount of impurities included in the purified protein (e.g., amount of polymers or host cell proteins), so that the objective protein can be purified with high purity.

Also, according to the purification method of the present invention, it is possible to purify a protein with high purity by including an amino acid as an ingredient of a buffer solution or a part thereof used in a chromatographic process. Therefore, a part of the steps of the conventional protein purification process can be eliminated, so that simplification of a protein purification device and economic benefits by increased yield are expected.

### BRIEF DESCRIPTION OF THE DRAWINGS

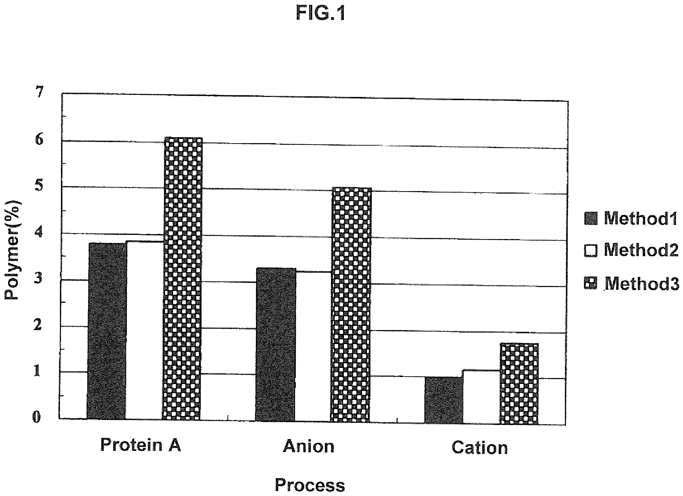

FIG. 1 shows a ratio of polymers contained in purification products after completion of each process in purification processes using a Protein A affinity chromatography column (MabSelect), an anion exchange chromatography column (Q Sepharose XL) and a cation exchange chromatography column (SP Sepharose FF), in which the vertical axis represents the ratio of the polymers.

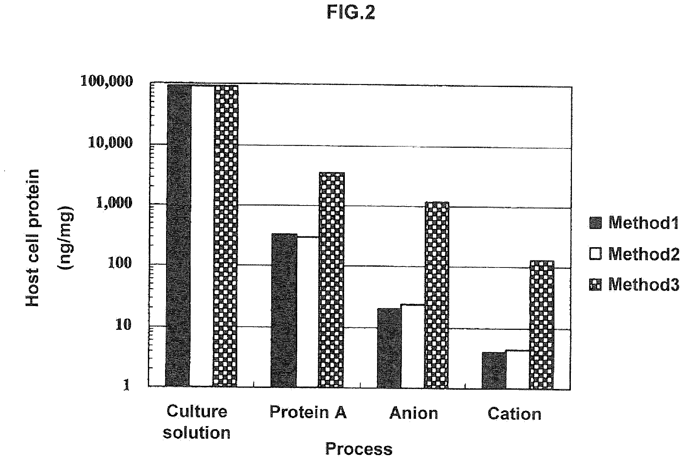

FIG. 2 shows a ratio of host cell proteins contained in purification products after completion of each process in purification processes using a Protein A affinity chromatography column (MabSelect), an anion exchange chromatography column (Q Sepharose XL) and a cation exchange chromatography column (SP Sepharose FF), in which the vertical axis represents the ratio of the host cell proteins.

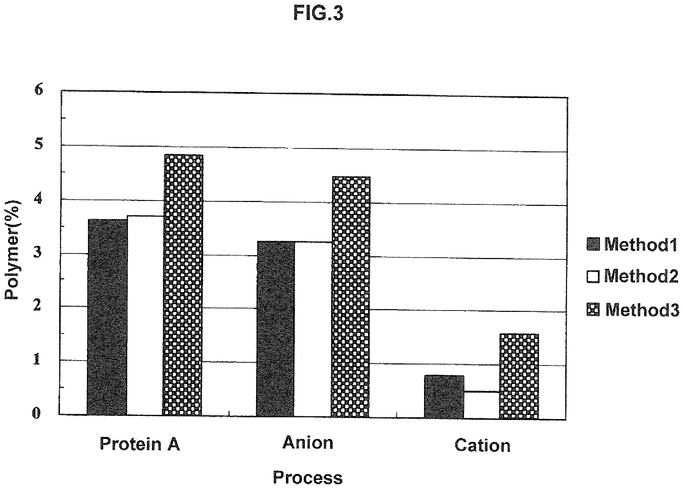

FIG. 3 shows a ratio of polymers contained in purification products after completion of each process in purification processes using a Protein A affinity chromatography column (MabSelect SuRe), an anion exchange chromatography column (Toyopearl Gigacap Q), and a cation exchange chromatography column (Fractogel SE HiCap), in which the vertical axis represents the ratio of the polymers.

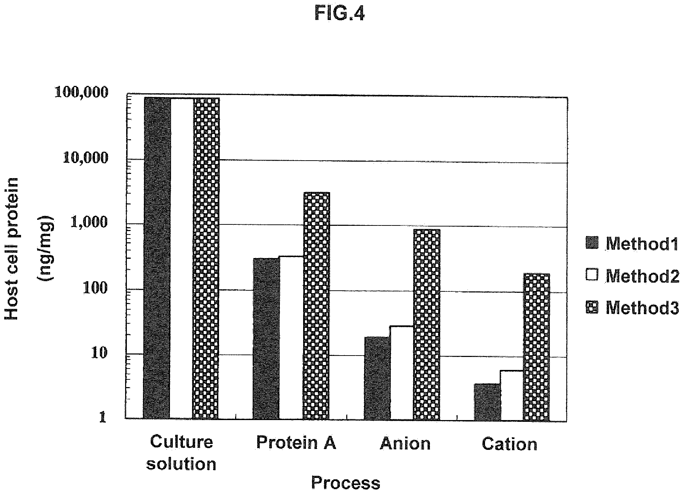

FIG. 4 shows a ratio of host cell proteins contained in purification products after completion of each process in purification processes using a Protein A affinity chromatography column (MabSelect SuRe), an anion exchange chromatography column (Toyopearl Gigacap Q), and a cation exchange chromatography column (Fractogel SE HiCap), in which the vertical axis represents the ratio of the host cell proteins.

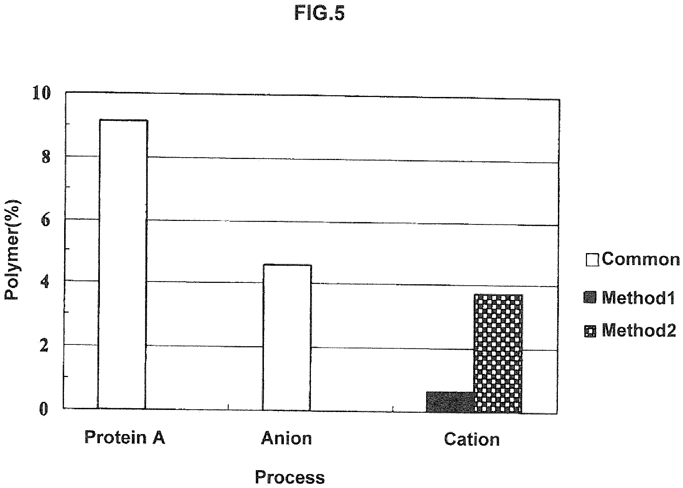

FIG. 5 shows a ratio of polymers contained in purification products after completion of each process in purification processes using a Protein A affinity chromatography column (MabSelect SuRe), an anion exchange chromatography column (Toyopearl Gigacap Q), and a cation exchange chromatography column (Fractogel SE HiCap), in which the vertical axis represents the ratio of the polymers (until the anion exchange chromatographic process, the same process is carried out in Method 1 and Method 2, and thus a ratio of the polymers is identical).

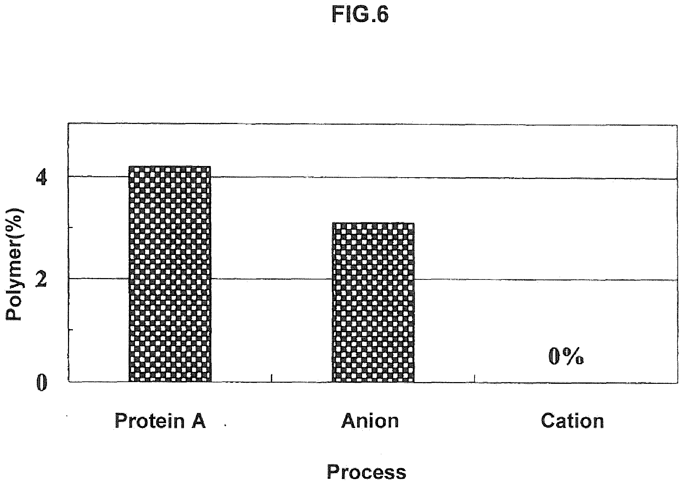

FIG. 6 shows a ratio of polymers contained in purification products after completion of each process in purification processes using a Protein A affinity chromatography column (MabSelect SuRe), an anion exchange chromatography column (Toyopearl Gigacap Q), and a cation exchange chromatography column (SP Sepharose FF), in which the vertical axis represents the ratio of the polymers.

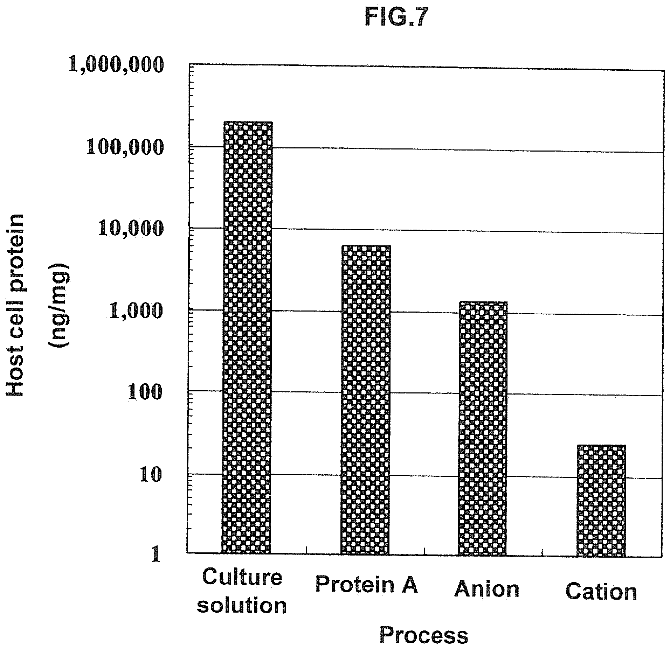

FIG. 7 shows a ratio of host cell proteins contained in purification products after completion of each process in purification processes using a Protein A affinity chromatography column (MabSelect SuRe), an anion exchange chromatography column (Toyopearl Gigacap Q), and a cation exchange chromatography column (SP Sepharose FF). The vertical axis represents the ratio of the host cell proteins.

### EMBODIMENTS FOR CARRYING OUT THE INVENTION

The present invention relates to a method as claimed for purifying a protein, including one or more chromatographic processes, in which an amino acid is included as an ingredient of a buffer solution or a part thereof used in at least one chromatographic process. The method of purifying a protein of the present invention is preferably used for purifying, in particular, monoclonal antibodies.

According to the purification method of the present invention, the amount of impurities (e.g., amount of polymers or host cell proteins) included in the purified protein, in particular, antibodies can be reduced and the objective protein can be purified with high purity, by including an amino acid as an ingredient of the buffer solution or a part thereof used in at least one chromatographic process.

The terms used in the present specification are intended to include the following definitions. Incidentally, the term "buffer solution" used in the present specification is synonymous with "buffer".

The substances purified by the purification method of the present invention are proteins. Among the proteins, antibodies are preferred, and monoclonal antibodies (mouse, chimeric, humanized, human antibodies, or modified antibodies thereof at the Fc region) are more preferred.

Examples of samples containing these proteins (hereinafter, referred to as the protein-containing sample) may include, for example, a culture supernatant. The culture supernatant may be, for example, obtained by culturing CHO cells, NS0 myeloma cells, hybridoma cells, or yeast cells, and then filtering them, or the like. If the objective protein is a monoclonal antibody, a content of the monoclonal antibody contained in the culture supernatant is preferably from 0.1 to 30 g/L, more preferably from 0.5 to 20 g/L, and much more preferably from 1 to 10 g/L.

Examples of the protein to be purified may also include polyclonal antibodies and monoclonal antibodies derived from serum, transgenic animal-derived milk or transgenic plant extract.

In the purification method of the present disclosure, examples of the amino acids included as an ingredient of the buffer solution or a part thereof used in the chromatographic process may include, for example, glycine (Gly), alanine (Ala), valine . (Val), leucine (Leu), isoleucine (Ile), serine (Ser), threonine (Thr), lysine (Lys), arginine (Arg), asparagine (Asn), aspartic acid (Asp), glutamine (Gin), glutamic acid (Glu), cysteine (Cys), methionine (Met), phenylalanine (Phe), tyrosine (Tyr), proline (Pro), tryptophane (Trp) or histidine (His), or dipeptides, oligopeptides, or polyamino acids thereof.

Among the above amino acids, glutamine, histidine, glutamic acid, glycine, arginine, and methionine are preferred. By including these amino acids as an ingredient of the buffer solution or a part thereof used in the chromatographic process, the purity of the protein to be purified is more improved.

Examples of the buffer solutions may include, for example, an equilibration buffer, a wash buffer and an elution buffer.

The purification method of the present invention includes one or more chromatographic processes. Examples of the chromatographic processes included in the method of the present invention may include a Protein A affinity chromatographic process, an anion exchange chromatographic process, a cation exchange chromatographic process, a gel filtration (size exclusion) chromatographic process, a hydrophobic chromatographic process, a hydroxyapatite chromatographic process, a mixed mode chromatographic process or the like.

The proteins are purified in much higher purity and yield by combinations of these chromatographic processes based on the knowledge of those skilled in the art. For example, as the purification method of monoclonal antibodies, it is preferable to carry out the cation chromatographic process subsequent to the Protein A affinity chromatographic process. Further, it is preferable to carry out the anion exchange chromatographic process subsequently

Additionally, as the purification of monoclonal antibodies, for example, it is preferable to carry out the cation exchange chromatographic process subsequent to the Protein A affinity chromatographic process and the anion exchange chromatographic process. By combining the chromatographic processes in this way, the monoclonal antibodies can be purified with higher purity and yield.

Instead of the cation exchange chromatography, the hydrophobic chromatography, the hydroxyapatite chromatography, or the mixed mode chromatography may be used. Further, the anion exchange chromatography may be carried out subsequent to the Protein A affinity chromatography and the cation exchange chromatography.

Furthermore, monoclonal antibodies may be purified without using the Protein A affinity chromatography. For example, combinations of at least two of the cation exchange chromatography, the anion exchange chromatography, the hydroxyapatite chromatography, and the mixed mode chromatography may be carried out.

In the purification method of the present invention, inclusion of the amino acid as an ingredient of the buffer solution or a part thereof in all chromatographic processes is not required, but the amino acid is included as an ingredient of the buffer solution or a part thereof in at least one chromatographic process.

Further, in each chromatographic process, the amino acid may be included as an ingredient of all of the equilibration buffer, the wash buffer, and the elution buffer; or a part thereof. The amino acid also may be included in only some of the buffers.

In the present invention, the phrase "including an amino acid as an ingredient of the buffer solution or a part thereof" means that the amino acid is the ingredient itself of the buffer solution or the amino acid is included in a part of the ingredients of the buffer solution. In the purification method of the present diclosure, a content of the amino acid included in the buffer solution used in at least one chromatographic process may be adjusted according to the types of the objective protein, the amino acid, the chromatographic process, and the like.

Specifically, a content of the amino acid in the equilibration buffer or the elution buffer of the disclosure is, for example, preferably from 10 to 1000 mM, and more preferably from 10 to 100 mM. Further, a content of the amino acid in the wash buffer is preferably from 0.5 to 3 M, and more preferably from 0.5 to 1 M.

In addition, from 0.1 to 2 M of an amino acid may be preferably included in the protein-containing sample loaded onto the chromatography, which makes it possible to reduce the level of impurities without a washing process.

Examples of the chromatographic processes, which preferably include the amino acid as an ingredient of the buffer solution or a part thereof, include the Protein A affinity chromatographic process and the cation exchange chromatographic process.

Specifically, in the following case of (1) or (2), for example, it is Referable to include glycine or threonine is as an ingredient of the buffer solution or a part thereof used in the Protein A affinity chromatographic process. Further, it is preferable to include an amino acid selected from glutamic acid, histidine and glutamine as an ingredient of the buffer solution or a part thereof used in the cation exchange chromatographic process.
(1) The case in which a Protein A chromatographic process is carried out, followed by carrying out a cation exchange chromatographic process, preferably, further followed by carrying out an anion exchange chromatographic process.
(2) The case in which a Protein A affinity chromatographic process and an anion exchange chromatographic process are carried out, followed by carrying out a cation exchange chromatographic process.

In combinations of a plurality of chromatographic processes, it is preferable to include the amino acid as an ingredient of the buffer solution or a part thereof in the final chromatographic process. This is because that there is an advantage that the exchange of the buffer solution is not needed when the amino acid included as an ingredient of the buffer solution or a part thereof in the final chromatographic process in the purification method of the present invention is identical to the amino acid included in the buffer solution used in the pharmaceutical formulation containing the protein purified by the purification method.

For example, in the combination of the cation exchange chromatography subsequent to the Protein A affinity chromatography and the anion exchange chromatography, the amino acid is preferably included as an ingredient of the buffer solution or a part thereof in the cation exchange chromatographic process as the final process.

Hereinafter, the Protein A affinity chromatographic process, the anion exchange chromatographic process or the cation exchange chromatographic process will be described.

### (Protein A affinity chromatographic process)

Examples of resins for the Protein A affinity chromatography may include MabSelect, Protein A Sepharose FF, MabSelect Xtra, and MabSelect SuRe, all manufactured by GE, Prosep vA Hicapacity, Prosep vA Ultra and Prosep Ultraplus, all manufactured by Millipore, and the like, but is not limited thereto. Examples of columns may include those having a length of from 2.5 to 40 cm.

When the protein-containing sample is loaded onto the Protein A affinity chromatography column, it is preferable that the Protein A affinity chromatography column is equilibrated with the equilibration buffer :in advance.

Examples of the equilibration buffers may include Tris, phosphoric acid, acetic acid, citric acid, succinic acid, tartaric acid, Bis-Tris, Bis-Tris propane and HEPS; 2-[4-(2-Hydroxyethyl)-1-piperazinyl] ethanesulfonic acid, MES; 2-Morpholinoethanesulfonic acid and the like, but is not limited thereto. Typically, the pH of the equilibration buffer is preferably from 5.0 to 9.0.

A washing process may be included prior to the elution of the objective protein. In the case of the Protein A affinity chromatography, the washing process using 5 column volumes of 10 mM Tris-1 M NaCl buffer solution (pH 7.0), or a buffer solution prepared by adding from 0.5 to 3 M of amino acid, or dipeptide, oligopeptide or polyamino acid thereof in 10 mM Tris is included prior to the elution of the objective protein, thereby removing non-specific adsorbed substances that are adsorbed onto the column resin.

Alternatively, Protein A affinity chromatography is preferably carried out by directly adding preferably from 0.1 to 2 M of amino acid, or dipeptide, oligopeptide or polyamino acid thereof to the protein-containing sample that is loaded onto the Protein A affinity chromatography.

Through this process, the level of impurities can be reduced without the washing process. Instead of the amino acid, it is preferable to add the same concentration of a salt such as NaCl, MgCl₂ or CaCl₂ to the protein-containing sample that is loaded onto the Protein A affinity chromatography, because the similar effect can be obtained.

Examples of the elution buffers used in the Protein A affinity chromatographic process may include a buffer solution containing from 10 to 100 mM of glycine, threonine or other amino acid; or dipeptide, oligopeptide or polyamino acid thereof, but is not limited thereto. Typically, the pH of the buffer solution is preferably from 2.5 to 4.5.

Typically, a linear velocity of loading the protein-containing sample and of passing the buffer solution onto/through the Protein A affinity chromatography column is preferably from 50 to 1000 cm/h.

### (Anion exchange chromatographic process)

Examples of resins for the anion exchange chromatography may include Q Sepharose XL, Q Sepharose FF, DEAE Sepharose FF, ANX Sepharose FF, Capto Q, and Capto DEAE, all manufactured by GE, TOYOPEARL GigaCap Q-650 and TOYOPEARL SuperQ-650, all manufactured by TOSOH, Fractogel DEAE, Fractogel TMAE, Fractogel TMAE Hicap and ESHMUNO Q, all manufactured by Merck, and the like, but is not limited thereto. A column having a proper length is prepared with these resins and is used. A length of the column is preferably from 2.5 to 40 cm. Furthermore, it is possible to use an anion exchange membrane.

Examples of the equilibration buffers of the anion exchange chromatography may include a buffer solution containing from 10 to 100 mM of histidine, or other amino acid, or dipeptide, oligopeptide or polyamino acid thereof, a Tris buffer solution, a buffer solution prepared by adding from 5 to 100 mM of threonine or other amino acid, or dipeptide, oligopeptide or polyamino acid thereof in from 10 to 100 mM Tris, and the like, but is not limited thereto. Typically, the pH of the buffer is preferably from 5.0 to 9.0.

Typically, a linear velocity of loading the protein-containing sample and of passing the buffer solution onto/through the anion exchange chromatography column is preferably from 50 to 1000 cm/h.

### (Cation exchange chromatographic process)

Examples of the cation exchange chromatography columns may include SP Sepharose FF, CM Sepharose FF, SP Sepharose XL, and Capto S, all manufactured by GE, Poros 50 HS and Poros 50 XS, all manufactured by Applied Biosystems, ESHMUNO S, Fractogel COO-, Fractogel SO3-, and Fractogel SE Hicap, all manufactured by Merck, TOYOPEARL GigaCap S-650 and TOYOPEARL GigaCap CM-650, all manufactured by TOSOH, and the like, but is not limited thereto. Typically, a length of the column is preferably from 2.5 to 40 cm.

Examples of the equilibration buffers of the cation exchange chromatography may include from 10 to 100 mM MES, acetic acid, citric acid, succinic acid, tartaric acid, or phosphoric acid buffer, a buffer solution containing from 10 to 100 mM of glutamic acid or other amino acid, or dipeptide, oligopeptide or polyamino acid thereof, and the like, but is not limited thereto. Typically, the pH of the buffer is preferably from 4.0 to 8.0.

Examples of the elution buffers used in the cation exchange chromatography may include a buffer solution prepared by adding arginine or other amino acid, or dipeptide, oligopeptide or polyamino acid thereof to from 10 to 100 mM MES, acetic acid, citric acid, succinic acid, or phosphoric acid buffer, or a buffer solution prepared by adding a salt such as NaCl to from 10 to 100 mM of glutamic acid or other amino acid, or dipeptide, oligopeptide or polyamino acid thereof, and a buffer solution containing from 10 to 1000 mM glutamic acid or other amino acid, or dipeptide, oligopeptide or polyamino acid thereof, but is not limited thereto. Typically, the pH of the buffer is preferably from 4.0 to 8.0.

Typically, a linear velocity of loading the protein-containing sample and of passing the buffer solution onto/through the cation exchange chromatography column is preferably from 50 to 1000 cm/h.

The elution process of the cation exchange chromatography may be any one of gradient elution accomplished by gradually increasing the concentration of the elution buffer, or stepwise elution using the elution buffers.

In considering industrial scale manufacture of medicinal products containing the protein purified by the purification method of the present invention as an active ingredient, and if the cation exchange chromatographic process is carried out as the final chromatographic process, use of the buffer solution identical to that in the pharmaceutical formulation of the medicinal products in the cation exchange chromatographic process provides a possibility that the necessary exchange of buffer is not required until the pharmaceutical formulation of the medicinal products is finally completed after the cation exchange chromatographic process.

In the purification method of the present invention, therefore, the amino acid included as an ingredient of the buffer solution or a part thereof used in the final chromatographic process, preferably used in the cation exchange chromatographic process is preferably identical to the amino acid included in the pharmaceutical formulation of the medicinal products.

That is, the present disclosure also provides the pharmaceutical formulation of the medicinal products including the protein purified by the purification method of the present invention as an active ingredient, and also including the amino acid identical to that included as an ingredient of the buffer solution or a part thereof used in the final chromatographic process, preferably used in the cation exchange chromatographic process, of the purification method of the present invention.

Furthermore, by using the amino acid identical to that included in the pharmaceutical formulation of the medicinal products in all chromatographic processes of the purification method of the present invention, more economic benefits are expected by reduction in the number of raw materials or simplification of the protein purification device,

Therefore, it is preferable that the amino acid included as an ingredient of the buffer solution or a part thereof in the chromatographic process of the purification method of the present invention is identical to that included in the pharmaceutical formulation of the medicinal products.

More preferably, glutamine, glutamic acid, histidine, arginine, or methionine is included in the buffer solution used in the chromatographic process of the purification method of the present disclosure and in the pharmaceutical formulation of the medicinal products including the protein purified by the purification method of the present invention as an active ingredient.

High purity monoclonal antibodies can be obtained by purifying the monoclonal antibodies according to the purification method of the present invention. With respect to a polymer, the "high purity" means that a ratio of the polymer in the purification product purified by the purification method of the present invention is preferably less than 4%, more preferably less than 3.5%,and much more preferably less than 1 %.

Additionally, with respect to a host cell protein, the it means that a content of the host cell-derived protein (host protein) in the purification product purified by the purification method of the present invention is preferably less than 1000 ng/mg (less than 1000 ng of host cell protein per 1 mg of protein), more preferably less than 100 ng/mg, and much more preferably less than 10 ng/mg.

It is possible to determine the concentration of the polymer or the host protein in the purification product by using the methods known to those skilled in the art: (e.g., ELISA for the host cell protein, or gel filtration HPLC for the polymer).

### EXAMPLES

### Example 1. Purification using MabSelect, Q Sepharose XL, and SP Sepharose FF

86 ml of the culture supernatant of CHO cells containing human monoclonal antibodies, which had been clarified by microfiltration, was applied to a Protein A affinity chromatography column (MabSelect, manufactured by GE, 10 mm ID×20 cm) (linear velocity: 500 cm/h) equilibrated with a 10 mM Tris buffer solution (pH 7.0). After application of the culture supernatant, the column was washed with 5 column volumes of an equilibration buffer (linear velocity: 500 cm/h).

Subsequently, the human monoclonal antibodies were eluted with 5 column volumes of an elution buffer (pH 3.2) shown in Table 1 (linear velocity: 500 cm/h). The eluate was neutralized to pH 7.0 with 1.5 M Tris.

The neutralized liquid was applied to an anion exchange chromatography column (Q Sepharose XL, manufactured by GE, 3 mm ID×20 cm) (linear velocity: 300 cm/h) equilibrated with an anion exchange buffer (pH 7.0) shown in Table 1. After completion of the application, 5 column volumes of the equilibration buffer were passed through the column. The column non-adsorbed fraction was pooled as a human monoclonal antibody eluate.

The pH of the Q Sepharose XL pooled liquid was adjusted to 5.0 with 1M citric acid, and then applied to a cation exchange chromatography column (SP Sepharose FF, manufactured by GE, 10 mm ID×20 cm) (linear velocity: 200 cm/h) equilibrated with a cation exchange equilibration buffer (pH 5.0) of Table 1. After completion of the application, the column was washed with 5 column volumes of the equilibration buffer (linear velocity: 200 cm/h).

Subsequently, human monoclonal antibodies were eluted with salt concentration gradient of gradually increasing salt concentration to 0.35 M (20 column volumes) using a cation exchange elution buffer (pH 4.5) shown in Table 1 (linear velocity: 200 cm/h).

**[Table 1]**

| Process | Buffer solution | Method 1 (use amino acid) | Method 2 (use amino acid) | Method 3 |
|---|---|---|---|---|
| Protein A | Elution buffer | 100mM glycine pH 3.2 | 100mM threonine pH 3.2 | 20mM citric acid pH 3.2 |
| Anion exchange | Equilibration buffer | 20mM histidine pH 7.0 | 10mM Tris pH 7.0, 10mM threonin | 10mM Tris pH 7.0 |
| Cation exchange | Equilibration buffer | 80mM MES pH 5.0 | 80mM glutamic acid pH 5.0 | 80mM MES pH 5.0 |
| | Elution buffer | 80mM MES pH 4.5, 0.35M arginine | 80mM glutamic acid pH 4.5, 0.35M NaCl | 80mM MES pH 4.5, 0.35M NaCl |

The purification results of the human monoclonal antibodies of the present Example are shown in FIGs. 1 and 2. As shown in FIGs. 1 and 2, it was found that Method 1 and Method 2 of including an amino acid in the buffer solution used in the chromatographic process are excellent in terms of purity (contents of polymers and host cell proteins), compared to Method 3 of including no amino acid in the buffer solution.

### Example 2. Purification using MabSelect SuRe, TOYOPEARL GigaCap Q, and Fractogel SE HiCap

86 ml of the culture supernatant of CHO cells containing human monoclonal antibodies, which had been clarified by microfiltration, was applied to a Protein A affinity chromatography column (MabSelect SuRe, manufactured by GE, 10 mm ID x 20 cm) (linear velocity: 500 cm/h) equilibrated with the 10 mM Tris buffer solution (pH 7.0). After application of the culture supernatant, the column was washed with 5 column volumes of the equilibration buffer (linear velocity: 500 cm/h).

Subsequently, the human monoclonal antibodies were eluted with 5 column volumes of the elution buffer (pH 3.2) shown in Table 1 (linear velocity: 500 cm/h). The eluate was neutralized to pH 7.0 with 1.5 M Tris.

The neutralized liquid was applied to an anion exchange chromatography column (TOYOPEARL GigaCap Q, manufactured by TOSOH, 3 mm ID×20 cm) (linear velocity: 500 cm/h) equilibrated with the anion exchange buffer (pH 7.0) shown in Table 1. After completion of the application, 5 column volumes of the equilibration buffer were passed through the column. The column non-adsorbed fraction was pooled as a human monoclonal antibody eluate.

The pH of the TOYOPEARL GigaCap Q pooled liquid was adjusted to 5.0 with 1M citric acid, and then applied to a cation exchange chromatography column (Fractogel SE Hicap, manufactured by Merck, 10 mm ID×20 cm) (linear velocity: 200 cm/h) equilibrated with the cation exchange equilibration buffer (pH 5.0) of Table 1.

After completion of the application, the column was washed with 5 column volumes of the equilibration buffer (linear velocity: 200 cm/h). Subsequently, human monoclonal antibodies were eluted with salt concentration gradient of gradually increasing salt concentration to 0.35 M (20 column volumes) using the cation exchange elution buffer (pH 4.5) shown in Table 1 (linear velocity: 200 cm/h).

The purification results of the human monoclonal antibodies by the present method are shown in FIGs. 3 and 4. As shown in FIGs. 3 and 4, it was found that Method 1 and Method 2 of including an amino acid in the buffer solution used in the chromatographic process are excellent in terms of purity (contents of polymers and host cell proteins), compared to Method 3 of including no amino acid in the buffer solution.

### Example 3. Purification by use of an amino acid in a cation exchange chromatographic process

245 ml of the culture supernatant of CHO cells containing human monoclonal antibodies, which had been clarified by microfiltration, was applied to a Protein A affinity chromatography column (MabSelect SuRe, manufactured by GE, 10 mm ID×20 cm) (linear velocity: 500 cm/h) equilibrated with the 10 mM Tris buffer solution (pH 7.0) shown in Table 2. After application of the culture supernatant, the column was washed with 5 column volumes of the equilibration buffer (linear velocity: 500 cm/h).

Subsequently, the human monoclonal antibodies were eluted with 5 column volumes of the elution buffer (100 mM glycine, pH 3.4) shown in Table 2 (linear velocity: 500 cm/h). The eluate was neutralized to pH 7 with 1 M NaOH.

The neutralized liquid was applied to an anion exchange chromatography column (Gigacap Q, manufactured by TOSOH, 3 mm ID×20 cm) (linear velocity: 500 cm/h) equilibrated with the anion exchange buffer (10 mM Tris buffer, pH 7.0) shown in Table 2. After completion of the application, 5 column volumes of the equilibration buffer were passed through the column. The column non-adsorbed fraction was pooled as a human monoclonal antibody eluate.

The pH of the Gigacap Q pooled liquid was adjusted to 5.0 with 1 M hydrochloric acid, and divided equally (each 16 mL). Then, cation exchange chromatography was carried out under the conditions of Method 1 (by use of amino acid) and Method 2, respectively.

The loading liquid was applied to a cation exchange chromatography column (Fractogel SE Hicap, manufactured by Merck, 10 mm ID×20 cm) (linear velocity: 200 cm/h) equilibrated with the cation exchange equilibration buffer (pH 5.0) of Table 2.

After completion of the application, the column was washed with 5 column volumes of the equilibration buffer (linear velocity: 200 cm/h). Subsequently, human monoclonal antibodies were eluted with gradient of gradually increasing the elution buffer shown in Table 2 (20 column volumes) using the cation exchange elution buffer (pH 5.0) (linear velocity: 200 cm/h).

**[Table 2]**

| Process | Buffer solution | Method 1 (use amino acid) | Method 2 |
|---|---|---|---|
| Protein A | Equilibration buffer | 10mM Tris pH 7.0 | |
| | Elution buffer | 100mM glycine pH 3.4 | |
| Anion exchange | Equilibration buffer | 10mM Tris pH 7.0 | |
| Cation exchange | Equilibration buffer | 20mM glutamic acid pH 5.0 | 20mM citric acid pH 5.0 |
| | Elution buffer | 600mM glutamic acid pH 5.0 | 600mM citric acid pH 5.0 |

The purification results of the human monoclonal antibodies by the present method are shown in FIG. 5. As shown in FIG.5, it was found that Method 1 of including the amino acid in the buffer solution used in the chromatographic process are excellent in terms of purity (content of polymers), compared to Method 2 of including no amino acid in the buffer solution.

### Example 4. Purification by sole use of a buffer solution using an amino acid (histidine) that is an ingredient of a buffer solution for the pharmaceutical formulation of the medicinal products

99 ml of the culture supernatant of CHO cells containing human monoclonal antibodies, which had been clarified by microfiltration, was applied to the Protein A affinity chromatography column (MabSelect SuRe, manufactured by GE, 10 mm ID×20 cm) (linear velocity: from 403 to 500 cm/h) equilibrated with the 10 mM histidine buffer solution (pH 7.0) shown in Table 3. After application of the culture supernatant, the column was washed with 5 column volumes of the equilibration buffer (linear velocity: 500 cm/h).

Subsequently, the human monoclonal antibodies were eluted with 5 column volumes of the elution buffer (50 mM histidine, pH 3.4) shown in Table 3 (linear velocity: 500 cm/h). The eluate was neutralized to pH 7 with 1 M NaOH.

The neutralized liquid was applied to an anion exchange chromatography column (TOYOPEARL GigaCap Q, manufactured by TOSOH, 10 mm ID×20 cm) (linear velocity: 500 cm/h) equilibrated with the anion exchange buffer (10 mM histidine, pH 7.0) shown in Table 3. After completion of the application, 5 column volumes of the equilibration buffer were passed through the column. The column non-adsorbed fraction was pooled as a human monoclonal antibody eluate.

The pH of the TOYOPEARL GigaCap Q pooled liquid was adjusted to 4.5 with 1 M hydrochloric acid, and then applied to a cation exchange chromatography column (SP Sepharose FF, manufactured by GE, 10 mm ID×20 cm) (linear velocity: 200 cm/h) equilibrated with a cation exchange equilibration buffer (50 mM histidine, pH 4.5) of Table 3.

After completion of the application, the column was washed with 5 column volumes of the equilibration buffer (linear velocity: 200 cm/h). Subsequently, human monoclonal antibodies were eluted with gradient of gradually increasing the elution buffer shown in Table 3 (20 column volumes) using a cation exchange elution buffer (250 mM histidine, pH 6.0) (linear velocity: 200 cm/h).

**[Table 3]**

| Process | Buffer solution | Method |
|---|---|---|
| Protein A | Equilibration buffer | 10mM histidine pH 7.0 |
| | Elution buffer | 50mM histidine pH 3.4 |
| Anion exchange | Equilibration buffer | 10mM histidine pH 7.0 |
| Cation exchange | Equilibration buffer | 50mM histidine pH 4.5 |
| | Elution buffer | 250mM histidine pH 6.0 |

The purification results of the human monoclonal antibodies by the present method are shown in FIGs. 6 and 7. As shown in FIGs. 6 and 7, the ratio of the polymer was less than 1% and the host cell protein was less than 50 ppm of in the purification product of the cation exchange chromatographic process. This purity is a quality level sufficient for use as medicinal products.

Further, it was found that the protein purified by the purification method of the present invention including the ammo acid in the buffer solution used in the chromatographic process is excellent in terms of purity (contents of polymer and host cell protein), compared to the method of including no amino acid in the buffer solution used in the chromatographic process, as in Method 3 of Table 1 that was examined in Examples 1 and 2 (see FIGs. 1 to 4).

For example, when the amino acid is included in the buffer solution, an amount of the host cell protein (see FIG. 7) in the purification product after the cation exchange chromatography column was 20% or less of that in the case of including no amino acid in the buffer solution (see Method 3 of FIGs. 2 and 4).

Further, as in the present Example, the antibody can be purified by sole use of a buffer solution containing one kind of amino acid, and the use of the same amino acid as the buffer ingredient of a pharmaceutical formulation give rise to a possibility of reducing the number of raw materials used in the purification or of eliminating the buffer exchanging process, and thus more economic benefits are expected by a reduction in the costs of raw materials and simplification of the protein purification device.

## Claims

1. A method for purifying a protein to reduce the amount of polymers and host cell proteins, comprising one or more chromatographic processes, wherein the one or more chromatographic processes comprises a Protein A affinity chromatographic process, and wherein glycine is included as the ingredient itself of the elution buffer used in the Protein A affinity chromatographic process, the content of the glycine in the elution buffer being 100 mM, and the pH of the elution buffer being from 3.2-3.4.

2. The purification method according to claim 1, wherein the one or more chromatographic processes further comprises a cation exchange chromatographic process.

3. The purification method according to claim 2, wherein an amino acid is included as an ingredient of a buffer solution or a part thereof used in the cation exchange chromatographic process.

4. The purification method according to any one of claims 1 to 3, which further comprises, as the chromatographic process, one or more chromatographic processes selected from an anion exchange chromatographic process, a gel filtration chromatographic process, a hydrophobic chromatographic process, a hydroxyapatite chromatographic process, and a mixed mode chromatographic process.

5. The purification method according to any one of claims 2 to 4, wherein the cation exchange chromatographic process is carried out subsequently to the Protein A affinity chromatographic process.

6. The purification method according to claim 5, wherein an anion exchange chromatographic process is further subsequently carried out.

7. The purification method according to claim 4, wherein the cation exchange chromatographic process is carried out subsequently to the Protein A affinity chromatographic process and an anion exchange chromatographic process.

8. The purification method according to any one of claims 2 to 7, wherein an amino acid included as an ingredient of a buffer solution or a part thereof used in the cation exchange chromatographic process is an amino acid selected from glutamic acid, histidine, and glutamine.

9. The purification method according to any one of claims 1 to 8, wherein a salt is added to a protein-containing sample that is loaded onto the Protein A affinity chromatography in the Protein A affinity chromatographic process.

10. The purification method according to any one of claims 2 to 9, wherein a ratio of host cell proteins in the purification product after completion of the cation exchange chromatographic process is less than 100 ng/mg.

11. The purification method according to any one of claims 2 to 10, wherein a ratio of polymers in the purification product after completion of the cation exchange chromatographic process is less than 1.5%.

12. A method for purifying a protein to reduce the amount of polymers and host cell proteins according to any one of claims 2 to 11, wherein an amino acid included in a pharmaceutical formulation of a medicinal product comprising the protein purified by the purification method according to any one of claims 2 to 11 as an active ingredient is identical to an amino acid included as an ingredient of the buffer solution or a part thereof used in the cation exchange chromatographic process according to any one of claims 2 to 11.

## Patentansprüche

1. Verfahren zur Aufreinigung eines Proteins, um die Menge an Polymeren und Wirtszellproteinen zu verringern, umfassend einen oder mehrere chromatographische Prozesse, wobei der eine oder die mehreren chromatographischen Prozess einen Protein A-Affinitätschromatographie-Prozess umfasst, und wobei Glycin als der Bestandteil selbst des Elutionspuffers enthalten ist, der in dem Protein A-Affinitätschromatographie-Prozess verwendet wird, wobei der Gehalt des Glycins im Elutionspuffer 100 mM ist und der pH-Wert des Elutionspuffers von 3,2 - 3,4 ist.

2. Aufreinigungsverfahren gemäß Anspruch 1, wobei der eine oder die mehreren chromatographischen Prozesse weiter einen Kationenaustauschchromatographie-Prozess umfasst.

3. Aufreinigungsverfahren gemäß Anspruch 2, wobei eine Aminosäure als ein Bestandteil einer Pufferlösung oder eines Teils davon, die/der in dem Kationenaustauschchromatographie-Prozess verwendet wird, enthalten ist.

4. Aufreinigungsverfahren gemäß irgendeinem der Ansprüche 1 bis 3, welches weiter umfasst, als den chromatographischen Prozess, einen oder mehrere chromatographische Prozesse, ausgewählt aus einem Anionenaustauschchromatographie-Prozess, einem Gelfiltrationschromatographie-Prozess, einem hydrophoben Chromatographie-Prozess, einem Hydroxyapatit-Chromatographie-Prozess und einem chromatographischen Prozess mit gemischtem Modus (mixed mode chromatographic process).

5. Aufreinigungsverfahren gemäß irgendeinem der Ansprüche 2 bis 4, wobei der Kationenaustauschchromatographie-Prozess nach dem Protein A-Affinitätschromatographie-Prozess durchgeführt wird.

6. Aufreinigungsverfahren gemäß Anspruch 5, wobei ein Anionenaustauschchromatographie-Prozess weiter danach durchgeführt wird.

7. Aufreinigungsverfahren gemäß Anspruch 4, wobei der Kationenaustauschchromatographie-Prozess nach dem Protein A-Affinitätschromatographie-Prozess und einem Anionenaustauschchromatographie-Prozess durchgeführt wird.

8. Aufreinigungsverfahren gemäß irgendeinem der Ansprüche 2 bis 7, wobei eine Aminosäure, die als ein Bestandteil einer Pufferlösung oder eines Teils davon enthalten ist, die/der in dem Kationenaustauschchromatographie-Prozess verwendet wird, eine Aminosäure, ausgewählt aus Glutaminsäure, Histidin und Glutamin, ist.

9. Aufreinigungsverfahren gemäß irgendeinem der Ansprüche 1 bis 8, wobei ein Salz zu einer proteinhaltigen Probe hinzugefügt wird, die in dem Protein A-Affinitätschromatographie-Prozess auf die Protein A-Affinitätschromatographie geladen wird.

10. Aufreinigungsverfahren gemäß irgendeinem der Ansprüche 2 bis 9, wobei ein Anteil an Wirtszellproteinen im Aufreinigungsprodukt nach Fertigstellung des Kationenaustauschchromatographie-Prozesses weniger als 100 ng/mg ist.

11. Aufreinigungsverfahren gemäß irgendeinem der Ansprüche 2 bis 10, wobei ein Anteil von Polymeren im Aufreinigungsprodukt nach Fertigstellung des Kationenaustauschchromatographie-Prozesses weniger als 1,5% ist.

12. Verfahren zur Aufreinigung eines Proteins, um die Menge an Polymeren und Wirtszellproteinen gemäß irgendeinem der Ansprüche 2 bis 11 zu verringern, wobei eine in einer pharmazeutischen Formulierung eines medizinischen Produkts, welches das durch das Aufreinigungsverfahren gemäß irgendeinem der Ansprüche 2 bis 11 aufgereinigte Protein als einen aktiven Bestandteil umfasst, enthaltene Aminosäure identisch zu einer Aminosäure ist, die als ein Bestandteil der Pufferlösung oder eines Teils davon, die/der in dem Kationenaustauschchromatographie-Prozess gemäß irgendeinem der Ansprüche 2 bis 11 verwendet wird, enthalten ist.

## Revendications

1. Procédé de purification d'une protéine pour réduire la quantité de polymères et de protéines de cellule hôte, comprenant un ou plusieurs processus chromatographiques, dans lequel les un ou plusieurs processus chromatographiques comprennent un processus chromatographique par affinité avec la protéine A, et dans lequel de la glycine est incluse en tant que l'ingrédient lui-même du tampon d'élution utilisé dans le processus chromatographique par affinité avec la protéine A, la teneur de la glycine dans le tampon d'élution étant de 100 mM, et le pH du tampon d'élution étant de 3,2 à 3,4.

2. Procédé de purification selon la revendication 1, dans lequel les un ou plusieurs processus chromatographiques comprennent en outre un processus chromatographique par échange de cations.

3. Procédé de purification selon la revendication 2, dans lequel un acide aminé est inclus en tant qu'ingrédient d'une solution tampon ou d'une partie de celle-ci utilisée dans le processus chromatographique par échange de cations.

4. Procédé de purification selon l'une quelconque des revendications 1 à 3, qui comprend en outre, en tant que processus chromatographique, un ou plusieurs processus chromatographiques sélectionnés parmi un processus chromatographique par échange d'anions, un processus chromatographique par filtration sur gel, un processus chromatographique hydrophobe, un processus chromatographique avec de l'hydroxyapatite, et un processus chromatographique en mode mixte.

5. Procédé de purification selon l'une quelconque des revendications 2 à 4, dans lequel le processus chromatographique par échange de cations est réalisé à la suite du processus chromatographique par affinité avec la protéine A.

6. Procédé de purification selon la revendication 5, dans lequel un processus chromatographique par échange d'anions est en outre réalisé à la suite.

7. Procédé de purification selon la revendication 4, dans lequel le processus chromatographique par échange de cations est réalisé à la suite du processus chromatographique par affinité avec la protéine A et d'un processus chromatographique par échange d'anions.

8. Procédé de purification selon l'une quelconque des revendications 2 à 7, dans lequel un acide aminé inclus en tant qu'ingrédient d'une solution tampon ou d'une partie de celle-ci utilisée dans le processus chromatographique par échange de cations est un acide aminé sélectionné parmi l'acide glutamique, l'histidine, et la glutamine.

9. Procédé de purification selon l'une quelconque des revendications 1 à 8, dans lequel un sel est ajouté à un échantillon contenant des protéines qui est chargé sur la chromatographie par affinité avec la protéine A dans le processus chromatographique par affinité avec la protéine A.

10. Procédé de purification selon l'une quelconque des revendications 2 à 9, dans lequel une proportion de protéines de cellule hôte dans le produit de purification après l'achèvement du processus chromatographique par échange de cations est inférieure à 100 ng/mg.

11. Procédé de purification selon l'une quelconque des revendications 2 à 10, dans lequel une proportion de polymères dans le produit de purification après l'achèvement du processus chromatographique par échange de cations est inférieure à 1,5 %.

12. Procédé de purification d'une protéine pour réduire la quantité de polymères et de protéines de cellule hôte selon l'une quelconque des revendications 2 à 11, dans lequel un acide aminé inclus dans une formulation pharmaceutique d'un produit médicinal comprenant la protéine purifiée par le procédé de purification selon l'une quelconque des revendications 2 à 11 en tant qu'ingrédient actif est identique à un acide aminé inclus en tant qu'ingrédient de la solution tampon ou d'une partie de celle-ci utilisée dans le processus chromatographique par échange de cations selon l'une quelconque des revendications 2 à 11.
